# EUROPÄISCHE PATENTSCHRIFT

(11) **EP 0 349 988 B1**
(45) Veröffentlichungstag und Bekanntmachung des Hinweises auf die Patenterteilung: **22.06.1994**
(21) Anmeldenummer: 89112215.2
(22) Anmeldetag: 04.07.1989
(51) Int. Cl.: G01N 33/536, G01N 33/546, G01N 33/543, G01N 33/58

(54) **Verfahren zur Bestimmung einer spezifisch bindefähigen Substanz**
Method to determine a specific substance capable of binding
Méthode pour déterminer une substance spécifique capable de lier

(30) Priorität: 05.07.1988 DE 3822750
(43) Veröffentlichungstag der Anmeldung: 10.01.1990
(73) Patentinhaber: BOEHRINGER MANNHEIM GMBH, 68298 Mannheim (DE)
(72) Erfinder: Schenk, Roland, Dr., D-8120 Weilheim (DE); Zdunek, Dietmar, Dr., D-8000 München 40 (DE)
(74) Vertreter: Huber, Bernhard, Dipl.-Chem.

(56) Entgegenhaltungen:
- EP-A- 0 138 297
- EP-A- 0 232 816
- EP-A- 0 308 242
- US-A- 4 829 011

## Beschreibung

Die Erfindung betrifft ein Verfahren zur Bestimmung einer spezifisch bindefähigen Substanz durch Inkubation der Probelösung mit mindestens drei Rezeptoren R₁, R₂ und R₃, von denen R₁ und R₂ miteinander bindefähig sind und R₃ mit der zu bestimmenden Substanz spezifisch bindefähig ist und Messung der bei der Reaktion auftretenden Agglutination sowie ein dazu geeignetes Reagenz.

In Körperflüssigkeiten und Geweben kommen sehr viele Substanzen vor, die mit einem spezifischen Bindungspartner bindefähig sind und als Parameter für bestimmte Erkrankungen oder den Gesundheitszustand des menschlichen Körpers dienen. Hierzu zählen unter anderem Haptene wie z. B. Hormone, Proteine wie Tumormarker, Proteinhormone und virale Proteine sowie Antikörper. Auch ist zur Überwachung einer medikamentösen Behandlung häufig die Bestimmung von Arzneistoffen im Blut erforderlich. Da diese Substanzen oft nur in sehr geringen Mengen vorkommen, verwendet man zu ihrem Nachweis Verfahren nach dem Prinzip des Immunoassays. Dazu gibt es viele Varianten. Die verschiedenen immunologischen Bestimmungsverfahren lassen sich in homogene und heterogene Verfahren einteilen. Bei den heterogenen Verfahren ist immer eine Festphasenreaktion beteiligt, um den gebundenen Anteil der markierten Komponenten von dem ungebundenen abzutrennen. Bei dieser Verfahrensart läßt sich die Markierung gut bestimmen, nachteilig ist jedoch, daß die heterogene Reaktion lange dauert.

Bei der homogenen Verfahrensvariante erfolgt keine Trennung von gebundener Markierung und ungebundener Markierung, so daß eine Differenzierung von gebundener und ungebundener Markierung durch andere Methoden erfolgen muß.

Hierzu gibt es verschiedene Möglichkeiten. So können beispielsweise konjugierte Enzyme als Markierung verwendet werden, die nur dann ihre Enzymaktivität erhalten, wenn sie an das zu bestimmende Hapten oder Antigen gebunden werden oder durch die zu bestimmende Substanz aktiviert werden. Eine weitere Möglichkeit besteht darin, als Markierung eine fluoreszierende Substanz zu verwenden, deren Fluoreszenz durch Bindung an die zu bestimmende Substanz entweder in einen anderen Wellenbereich verschoben wird oder deren Polarisation geändert wird.

Die Nachteile dieser bekannten Verfahren liegen insbesondere darin, daß die Probe häufig den Test störende Komponenten enthält, was eine Probenvorbehandlung zur Beseitigung dieser Substanzen erforderlich macht. Außerdem ist eine aufwendige Optimierung für jeden Parameter erforderlich, z. B. müssen die Enzyme parameterabhängig modifiziert werden.

Weiterhin ist aus EP-A-0 079 962 ein Verfahren bekannt, bei dem die das zu bestimmende Hapten enthaltende Lösung mit haptenbeschichteten Latexpartikeln oder haptenbeschichtetem Albumin in Kontakt gebracht wird. Durch Zugabe von mit den Hapten bindefähigen Antikörpern erfolgt eine Agglutinationsreaktion. Da das an die Latexteilchen bzw. an das Albumin gebundene Hapten mit dem in der Probe enthaltenen Hapten konkurriert, ist die Agglutinationsreaktion umso geringer, je mehr Hapten in der Probe enthalten ist. Der Nachteil dieses Verfahrens liegt darin, daß für jede zu bestimmende Substanz spezielle Teilchen zur Verfügung gestellt werden müssen und jeder Parameter individuell optimiert werden muß.

Ein weiteres Verfahren zum Nachweis von Proteinen, das auf der Auswertung einer Agglutinationsreaktion beruht, ist aus DE-A-27 49 956 bekannt. Dabei werden Antikörper gegen die zu bestimmende Substanz direkt an die agglutinierbaren Teilchen gebunden. Durch diese Bindung kann jedoch die Reaktivität der Antikörper beeinträchtigt werden. Außerdem ist ein derartiges Bestimmungsverfahren anfällig für Störungen durch Rheumafaktoren.

Nachteil aller bekannten kompetitiven homogenen Agglutinations-Immunoassays ist es, daß die Rohstoffe sehr aufwendig parameterspezifisch optimiert werden müssen. Bei all diesen Tests bestehen einander entgegengesetzte Anforderungen für eine optimale Differenzierung und optimale Empfindlichkeit, da einerseits die Konzentration des partikelförmigen Reagenzes limitiert sein soll, damit die Konkurrenzreaktion mit der Probe sinnvoll möglich ist und andererseits das partikelförmige Reagenz hochkonzentriert und hochmarkiert sein soll, um eine genügende Signaländerung pro Zeiteinheit zu erzielen. Die Abstimmung dieser Anforderungen führt zu eingeschränkter Empfindlichkeit und Störanfälligkeit, die oft nur durch spezifische Probenvorbehandlung beseitigt werden können.

Aufgabe der vorliegenden Erfindung war es daher, ein homogenes Bestimmungsverfahren zur Verfügung zu stellen, das den Nachweis von Substanzen mit hoher Empfindlichkeit und Genauigkeit ermöglicht und die oben beschriebenen Nachteile nicht hat.

Diese Aufgabe wird gelöst durch ein Verfahren zur Bestimmung einer spezifisch bindefähigen Substanz durch Inkubation mit drei Rezeptoren R₁,R₂ und R₃, von denen R₁ und R₂ miteinander bindefähig sind und R₃ mit der zu bestimmenden Substanz spezifisch bindefähig ist und Messung der bei der Reaktion auftretenden Agglutination, welches dadurch gekennzeichnet ist, daß als Rezeptor R₁ ein Konjugat aus einem Partner eines spezifisch bindenden Paares P und einer Substanz S, die der zu bestimmenden Substanz entspricht oder ein Derivat davon ist und mindestens ein Epitop der zu bestimmenden Substanz aufweist, wobei R₁ nur eine Bindungsstelle für R₂ besitzt, als R₂ ein Rezeptor, der mindestens zwei Bindungsstellen für P aufweist aber kein Erythrozyt ist und als R₃ ein Rezeptor, der mindestens zwei Bindungsstellen aufweist, von denen mindestens eine spezifisch mit einem Epitop der zu bestimmenden Substanz bzw. von S bindet, verwendet wird und R₂ gleichzeitig mit den Rezeptoren R₁ und R₃ oder nach einem bestimmten Zeitraum zugegeben wird oder R₂ und R₃ vorgemischt werden und nach Zugabe der Probe R₁ zugesetzt wird.

Das erfindungsgemäße Verfahren ist geeignet zur Bestimmung praktisch aller in Körperflüssigkeiten oder Gewebeextrakten nachzuweisenden zu einer spezifischen Bindung befähigen Substanzen, wobei niedrigkonzentrierte Substanzen ebenso gut nachweisbar sind wie hochkonzentrierte. Die Empfindlichkeit und Genauigkeit des Verfahrens ist gegenüber den bisher bekannten Verfahren verbessert. Die Erfindung bietet die Möglichkeit, mit einfachen Reagenzien schnell und zuverlässig Bestimmungen durchzuführen.

Das Verfahren ist insbesondere geeignet zur Bestimmung von monovalenten spezifisch bindefähigen Substanzen. Als monovalent wird dabei eine Substanz bezeichnet, die für einen spezifisch bindefähigen Partner nur eine Bindungsstelle besitzt. Als Beispiele sind hier zu nennen Haptene, z. B. Arzneistoffe. Ebenso können Substanzen bestimmt werden, die mehrere Bindungsstellen für spezifisch bindefähige Partner besitzen wie z.B. Proteinhormone wie HCG oder TSH, Antigene und Proteine, Tumormarker wie CEA, virale Proteine und Antikörper.

Unter Epitop wird in der Beschreibung der vorliegenden Erfindung eine Bindungsstelle verstanden, die eine spezifische Bindung mit einer anderen Substanz eingehen kann. Beispiele für Epitope sind antigene Determinanten auf Antigenen und Haptene oder aber auch spezifische Bindungsstellen auf Proteinen.

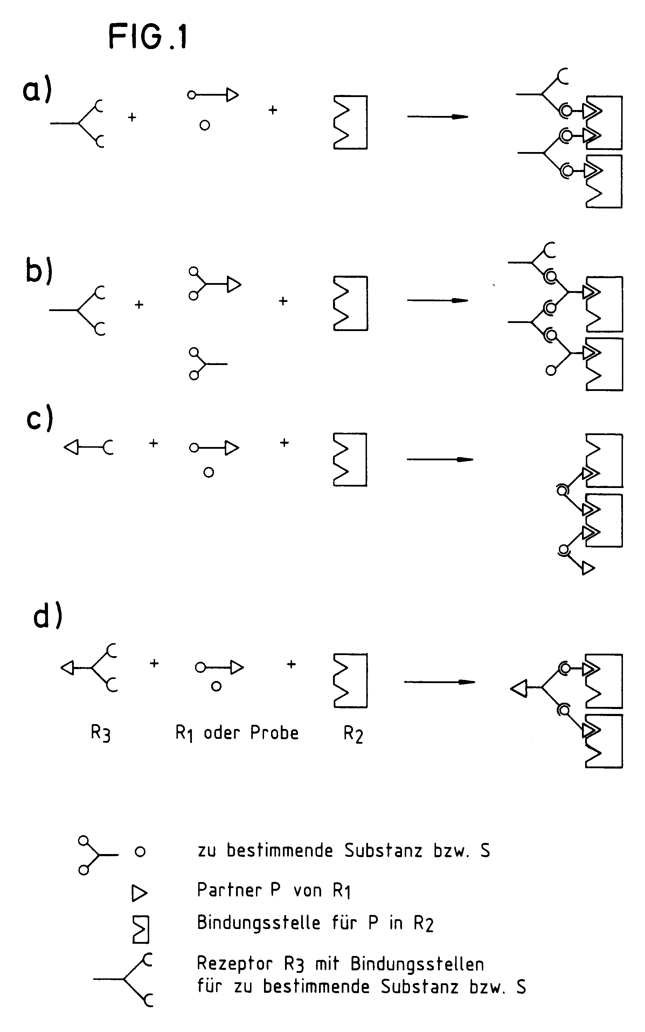

Die Probelösung wird dazu mit drei Rezeptoren R₁, R₂ und R₃ inkubiert. Die Rezeptoren R₁ und R₂ sind dabei miteinander bindefähig, und R₃ ist mit der zu bestimmenden Substanz spezifisch bindefähig. Verschiedene Reaktionsprinzipien, die mit dem erfindungsgemäßen Verfahren durchgeführt werden können, sind in der Fig. 1 dargestellt. Eine bevorzugte Verfahrensvariante ist die als 1a bezeichnete Durchführungsform. Dabei wird einer Probelösung Rezeptor R₁, der ein Konjugat aus einem Partner eines spezifisch miteinander bindenden Paares P und der zu bestimmenden Substanz S ist und nur eine Bindungsstelle für R₂ aufweist, sowie R₃, der ein mit der zu bestimmenden Substanz spezifisch bindefähiger Rezeptor ist, zugegeben. In der Lösung konkurrieren dann der Teil S von R₁ und die zu bestimmende Substanz um die Bindung an R₃. Ist R₃ beispielsweise ein bivalenter Antikörper, so bilden sich die folgenden Komplexe:
- R₃,: an dessen beiden Paratopen jeweils R₁ über S gegebunden ist;
- R₃,: an dessen beiden Paratopen die zu bestimmende Substanz gebunden ist, und
- R₃,: an dessen einem Paratop die zu bestimmende Substanz und an dessen anderem Paratop R₁ über S gebunden ist.

Gleichzeitig mit den anderen beiden Rezeptoren oder nach einem bestimmten Zeitraum wird der Rezeptor R₂ zugegeben, der mindestens zwei und bevorzugt eine Vielzahl Bindungsstellen für P aufweist aber kein Erythrozyt ist. Somit kommt es zur Bindung des Rezeptors R₁ über P an Rezeptor R₂. Von den in der Lösung vorhandenen Komplexen können nur die Komplexe, die mindestens einen Rezeptor R₁ gebunden aufweisen, an R₂ binden, und nur die, bei denen an R₂ zwei Rezeptoren R₁ gebunden sind, können eine Agglutination erzeugen, die wieder eine photometrisch detektierbare Trübung bzw. Trübungsänderung verursacht. Je mehr von der zu bestimmenden Substanz in der Lösung enthalten ist, desto weniger R₁ wird gebunden, desto weniger Agglutination tritt auf und desto geringer ist die Trübungszunahme. Somit ist der Umfang der Agglutination ein indirektes Maß für die zu bestimmende Substanz. Dieser kann über eine Eichkurve ausgewertet werden.

Die Durchführungsvariante b) dient zum Nachweis bivalenter Substanzen, z.B. Antikörper. Das Prinzip ist dabei das gleiche wie bei Variante a). Auch hier können wiederum nur die Komplexe, bei denen an den beiden Bindungsstellen von R₃ jeweils ein R₁ gebunden ist, eine Agglutination bewirken.

Bei der Variante c) wird als R₃ ein Konjugat aus einem mit der zu bestimmenden Substanz spezifisch bindefähigen Rezeptor und einem Partner eines spezifisch bindenden Paares verwendet. Bei Inkubation der Probelösung mit R₁ und R₃ konkurrieren wiederum die zu bestimmende Substant und R₁ um die Bindung an R₃. Dabei kann entweder ein Rezeptor R₁ oder ein Molekül der zu bestimmenden Substanz an R₃ binden. Nach Inkubation mit R₂ bindet der Anteil P des Rezeptors R₃ an R₂. Ist an der anderen Bindungsstelle von R₃ die zu bestimmende Substanz gebunden, so ist keine Vernetzung über R₂ möglich; ist jedoch an der anderen Bindungsstelle von R₃ das Konjugat R₁ gebunden, so liefert der Rezeptor R₁ eine zweite Bindungsstelle für R₂, und es kommt zur Vernetzung. Auch bei dieser Durchführungsform wird umso weniger R₁ gebunden, je mehr von der zu bestimmenden Substanz in der Lösung vorhanden ist, und dementsprechend ist die Agglutinationsbildung dann geringer.

Die Durchführungsform d) ist eine Variante der Durchführungsform a), wobei jedoch als Rezeptor R₃ ein Rezeptor verwendet wird, der außer den beiden Bindungsstellen für die zu bestimmende Substanz bzw. S noch eine weitere Bindungsstelle in Form von P aufweist. Bei Inkubation der Probelösung mit R₁ und R₃ bilden sich wieder dieselben Komplexe wie bei Variante a). Im Unterschied zu Variante a) können jedoch sowohl die Rezeptoren R₃, an deren beiden Paratopen R₁ gebunden ist, als auch die Rezeptoren R₃, bei denen an einem Paratop R₁ und am anderen die zu bestimmende Substanz gebunden ist, eine Agglutination bewirken. Diese Ausführungsform ist daher besonders geeignet zum Nachweis von Substanzen, die sehr hoch konzentriert sind.

Für das erfindungsgemäß definierte Verfahrensprinzip gibt es also viele Durchführungsvarianten. In jedem Fall sind mindestens drei Rezeptoren erforderlich. Die zu bestimmende Substanz kann jede zu einer spezifischen Bindung fähige Substanz sein und insbesondere wie oben definiert ein Hapten, monovalentes, bivalentes oder polyvalentes Antigen, Antikörper oder Protein sein.

Als erster Rezeptor R₁ wird ein Konjugat verwendet, das aus einem Partner eines spezifisch bindenden Paares P und einer Substanz S, die der zu bestimmenden Substanz entspricht oder ein Derivat davon ist und mindestens ein Epitop der zu bestimmenden Substanz aufweist, besteht und nur eine Bindungsstelle für R₂ besitzt. Spezifisch miteinander bindende Paare sind an sich bekannt. Geeignete Bindungspaare (P-R₂) sind insbesondere Biotin-Streptavidin bzw. Avidin; Hapten-Antikörper; Antigen-Antikörper; Konkavalin-Antikörper; Zucker-Lectin; Hapten-Bindeprotein, z.B. Thyroxin bindendes Globulin und Thyroxin oder Oligopeptid-Antikörper.

Besonders bevorzugt wird als bindendes Paar Streptavidin bzw. Avidin-Biotin eingesetzt. Besonders bevorzugt enthält der Rezeptor R₁ Biotin.

Der Anteil S des Rezeptors R₁ kann bevorzugt der unveränderten zu bestimmenden Substanz entsprechen. Es kann auch ein Derivat der zu bestimmenden Substanz bzw. ein Teil der zu bestimmenden Substanz wie beispielsweise ein Proteinepitop verwendet werden. Wesentlich ist nur, daß der Anteil S mit R₃ bindefähig ist, wobei es nicht unbedingt erforderlich ist, daß S und die zu bestimmende Substanz mit der gleichen Bindungsstärke an R₃ binden.

Die Herstellung der Konjugate erfolgt in an sich bekannter Weise (z. B. analog Eur. J. Biochem. 131 (1980) 333-338).

Der zweite für das erfindungsgemäße Verfahren erforderliche Rezeptor R₂ hat mindestens zwei Bindungsstellen für P und weist vorzugsweise eine Vielzahl der zu P komplementären Partner eines spezifisch bindenden Paares auf. Der Rezeptor R₂ vermittelt die Agglutination des sich bei der Reaktion bildenden Komplexes. Da eine Vielzahl dieser anderen Partner des spezifisch bindefähigen Paares im Reaktionssystem vorhanden sind, führt ein natürlich in der Probelösung vorhandener geringer Gehalt von mit diesem Partner bindefähiger Substanz nicht zu Störungen. Der Rezeptor R₂ kann eine Substanz sein, die bereits natürlicherweise mehrere Bindungsstellen für P besitzt, beispielsweise Streptavidin oder Antikörper. Rezeptor R₂ kann polyvalent sein und eine Vielzahl von Bindungsstellen für P aufweisen oder ein Polymer der zu P komplementären Partner des spezifisch bindenden Paares sein, wie z.B. Polystreptavidin. Dabei sind dann die einzelnen Partner miteinander entweder direkt verbunden oder aber über Brücken miteinander verknüpft. Verfahren zur Herstellung derartiger Polymere sind dem Fachmann bekannt und bedürfen keiner näheren Erläuterung.

In einer weiteren Ausführungsform besteht der Rezeptor R₂ aus einem Trägermaterial, an das eine Vielzahl der zu P komplementären spezifisch bindenden Partner gebunden sind. Als Trägermaterialien können Partikel verwendet werden, die üblicherweise eine Größe von 50 bis 1000 nm haben. Geeignete Materialien sind Polystyrol, feinverteiltes Siliziumdioxid, oder vernetztes Albumin. Die Beschichtung dieser Teilchen mit dem spezifisch bindenden Partner erfolgt nach den dem Fachmann geläufigen Methoden. Verfahren davon sind beispielsweise in EP-B-0 073 611 und US-A-4 703 018 beschrieben.

Die so beschichteten bzw. polymerisierten Rezeptoren R₂ sind universell für das erfindungsgemäße Verfahren einsetzbar und demzufolge nicht parameterspezifisch.

Wesentlich für die Durchführung des erfindungsgemäßen Verfahrens ist es, daß der Rezeptor R₁ nur eine Bindungsstelle für R₂ besitzt, d. h. daß jeder Rezeptor R₁ nur mit einem R₂ reagieren kann. Dies ist eine wesentliche Voraussetzung, da ansonsten R₂ allein durch den Rezeptor R₁ vernetzt werden könnte und damit eine Agglutination bewirkt würde, die nicht auf die nachzuweisende Substanz S zurückzuführen ist.

Die dritte erfindungswesentliche Komponente ist der Rezeptor R₃, der mindestens zwei Bindungsstellen aufweist, von denen mindestens eine spezifisch mit einem Epitop der zu bestimmenden Substanz bzw. von S bindet. Dieser Rezeptor wird nach der jeweils zu bestimmenden Substanz ausgewählt. Hier sind eine Vielzahl von Rezeptoren geeignet. Zur Bestimmung von Haptenen, Proteinen, DNA oder Zucker ist es besonders vorteilhaft, Antikörper oder sonstige Rezeptoren wie z. B. natürlich vorkommende Bindeproteine, wie Thyroxin-bindendes Globulin gegen diese Substanzen oder deren Fragmente zu verwenden. Zur Bestimmung von DNA ist es ebenfalls vorteilhaft, eine komplementäre DNA als Rezeptor R₃ zu verwenden.

Wenn die zu bestimmende Substanz nur eine einzige Bindungsstelle für R₃ aufweist, so weist in einer bevorzugten Ausführungsform der Rezeptor R₃ neben der einen Bindungsstelle für das Epitop der zu bestimmenden Substanz bzw. S eine davon verschiedene Bindungsstelle für einen Partner eines spezifisch bindenden Paares in Form des ebenfalls für R₁ verwendeten Partners P auf. R₃ ist dann bevorzugt entweder ein Konjugat aus einem Partner P eines spezifisch bindenden Paares und einer Substanz, die eine einzige Bindungsstelle aufweist, die spezifisch mit einem Epitop der zu bestimmenden Substanz bzw. von S bindet, besonders bevorzugt ein Konjugat aus einem mit einem Epitop der zu bestimmenden Substanz bzw. von S spezifisch bindenden Fab-Fragment und P oder ein Konjugat aus einem Partner P eines spezifisch bindenden Paares und einem Rezeptor, der zwei Bindungsstellen für das Epitop der zu bestimmenden Substanz bzw. S aufweist.

Rezeptor R₃ kann ebenso zwei gleiche Bindungsstellen aufweisen, die mit einem Epitop der zu bestimmenden Substanz bzw. S binden. Geeignet als Rezeptor R₃ sind beispielsweise monoklonale oder polyklonale Antikörper, Fab₂-Fragmente oder Konjugate aus Fab-Fragmenten und Partnern eines spezifisch bindenden Paares. Wenn der Rezeptor R₃ mehr als zwei Bindungsstellen aufweisen soll, können Konjugate von Antikörpern, F(ab)₂- oder F(ab′)₂-Fragmenten mit einem Partner P verwendet werden. Hier sind insbesondere biotinylierte Antikörper und Antikörperfragmente, die mit der zu bestimmenden Substanz spezifisch bindefähig sind, geeignet.

In einer weiteren Ausführungsform wird Rezeptor R₃ erst während der Bestimmung gebildet, z. B. aus einem Antikörper gegen TBG und zwei Molekülen TBG oder einem Antikörper gegen Fab-Fragmente und zwei Fab-Fragmenten.

Das Verfahren kann in einer oder mehr Stufen durchgeführt werden. Die Auswertung erfolgt durch Messung des Umfangs der Agglutination. Verfahren hierzu sind bekannt. Geeignet ist beispielsweise die photometrische Trübungsmessung, die Streulichtmessung durch Nephelometrie, Particle Counting oder Photon-Korrelations-Spektroskopie (PCS).

Da jeder der Rezeptoren und auch die zu bestimmende Substanz jeweils nur spezifisch mit dem für ihn bestimmten Reaktionspartner reagieren kann, ist es möglich, alle Rezeptoren und die Probe zusammen zu inkubieren und das Verfahren einstufig durchzuführen. Dies ist besonders vorteilhaft bei der Durchführung des Verfahrens in einem Analysenautomaten.

Für den Nachweis von sehr niedrigkonzentrierten oder sehr hochkonzentrierten Substanzen sind mehrstufige Verfahrensvarianten bevorzugt. Beim Nachweis von in der Probelösung hochkonzentriert vorliegenden Substanzen wird die im folgenden beschriebene Ausführungsform bevorzugt. Dabei wird zuerst Rezeptor R₁ mit Rezeptor R₃ vorreagiert. Dabei reagieren der Anteil S des Rezeptors R₁, der der zu bestimmenden Substanz entspricht, und R₃ miteinander. Anschließend wird die Probelösung zugegeben. Die in dieser Probelösung vorhandene zu bestimmende Substanz verdrängt nun bereits gebundenen Rezeptor R₁ aus der Bindung. Da in der Probelösung der Anteil an zu bestimmender Substanz hoch ist, findet diese Verdrängungsreaktion ausreichend schnell statt. Anschließend wird Rezeptor R₂ zugegeben und dann die Trübungszunahme gemessen. Diese Art der Testdurchführung ist zwar unempfindlicher, für hochkonzentrierte Proben jedoch sehr gut geeignet.

Eine weitere bevorzugte Ausführungsform des erfindungsgemäßen Verfahrens ist zum Nachweis sehr niedrigkonzentrierter Substanzen geeignet. Als Beispiele sind Hormone und Arzneistoffe zu nennen. Hierbei wird zuerst Rezeptor R₂ mit Rezeptor R₃ vorgemischt und anschließend die Probe zugegeben. Dabei reagiert in der Probe vorhandene zu bestimmende Substanz mit dem Rezeptor R₃. Eine Agglutinierung kann noch nicht stattfinden, da der die Bindung an den Rezeptor R₂ vermittelnde Rezeptor R₁ noch fehlt. Anschließend wird Rezeptor R₁ zugegeben, der mit dem Rezeptor R₃ und mit dem Rezeptor R₂ bindefähig ist. Auch hier wird die Trübungszunahme nach einem bestimmten Zeitraum gemessen. Diese Durchführungsvariante ist sehr empfindlich.

Für mittelkonzentrierte Parameter ist ebenso eine voll kompetitive Testdurchführung geeignet. In diesem Fall werden gleichzeitig Rezeptor R₁, Rezeptor R₃ und Probenlösung inkubiert. Dabei konkurrieren die zu bestimmende Substanz und der im Konjugat des Rezeptors R₁ enthaltene Anteil S um die Bindung an Rezeptor R₃. Nach Zugabe von Rezeptor R₂ wird dann wiederum die Agglutination gemessen.

Ebenfalls geeignet ist diese Variante des erfindungsgemäßen Verfahrens zum Nachweis von Haptenen die mit einem für sie spezifischen Protein gekuppelt werden können, wobei das Bindeprotein nur eine Bindungsstelle für das Hapten aufweist. Ein Beispiel ist Thyroxin, das spezifisch von Thyroxin bindendem Protein gebunden wird.

Die Durchführung aller Verfahrensvarianten erfolgt vorzugsweise in einer gepufferten Lösung. Puffersysteme für diese Verfahren sind an sich bekannt. Besonders geeignet sind hierfür GOOD-Puffer und Phosphatpuffer.

Erfindungsgemäß wird ein Verfahren zur Verfügung gestellt, das einfach und schnell durchzuführen ist und konzentrationsabhängig von der zu bestimmenden Substanz ein Meßsignal liefert. Da das für die immunologische Kompetition zuständige System und das signalbildende System getrennt sind, wird erfindungsgemäß die Empfindlichkeit des Nachweises erhöht. Dadurch können mit einfachen Reagentien sogar niedrigkonzentrierte Haptene schnell und zuverlässig quantitativ bestimmt werden.

Ein weiterer Gegenstand der vorliegenden Erfindung ist ein Reagenz zur Bestimmung von spezifisch bindefähigen Substanzen, welches dadurch gekennzeichnet ist, daß es Rezeptor R₁, der ein Konjugat aus einem Partner eines spezifisch bindenden Paares P und einer Substanz S, die der zu bestimmenden Substanz entspricht oder ein Derivat davon ist und mindestens ein Epitop der zu bestimmenden Substanz aufweist, wobei R₁ nur eine Bindungsstelle für R₂ besitzt, Rezeptor R₂, der mindestens zwei Bindungsstellen für P aufweist aber kein Erythrozyt ist und Rezeptor R₃, der mindestens zwei Bindungsstellen aufweist, von denen mindestens eine spezifisch mit einem Epitop der zu bestimmenden Substanz bzw. von S bindet, enthält.

Das erfindungsgemäße Reagenz kann in einer bevorzugten Ausführungsform die einzelnen Rezeptoren R₁, R₂ und R₃ physikalisch getrennt voneinander enthalten. In einer weiteren Ausführungsform können die Rezeptoren R₁ und R₃ vorgemischt werden und das Reagenz enthält dann die Mischung von R₁ und R₃ und Rezeptor R₂ physikalisch getrennt davon. Ebenso ist es möglich, die Rezeptoren R₂ und R₃ vorzumischen, wobei dann das Reagenz eine Mischung von R₂ und R₃ sowie physikalisch getrennt davon R₁ enthält.

Dieses Reagenz ist zur Bestimmung einer Vielzahl von Parametern in Körperflüssigkeiten und Gewebeextrakten geeignet.

In einer bevorzugten Ausführungsform enthält das Reagenz zusätzlich Puffersubstanzen. Besonders bevorzugt enthält es Phosphatpuffer oder GOOD-Puffer.

Die Erfindung wird durch die Figur und die Beispiele erläutert.
- Fig. 1: zeigt ein Schema für die Reaktionsprinzipien verschiedener bevorzugter Ausführungsformen des erfindungsgemäßen Verfahrens.

In allen gezeigten Varianten weist Rezeptor R₂ zwei Bindungsstellen für P auf. Als Rezeptor R₁ wird jeweils ein Konjugat aus der zu bestimmenden Substanz und einem Partner P eingesetzt.

Fig. 1a zeigt eine Variante, die zum Nachweis von Substanzen geeignet ist, die nur eine Bindungsstelle für Rezeptor R₃ haben.

Als Rezeptor R₃ wird hier ein bezüglich der zu bestimmenden Substanz bivalenter Rezeptor verwendet.

Variante 1b) dient zur Bestimmung von bivalenten Substanzen (z.B. Antikörper). Als Rezeptor R₃ wird ein mit der zu bestimmenden Substanz spezifisch bindefähiger bivalenter Rezeptor verwendet.

Variante 1c) ist geeignet zum Nachweis von monovalenten Substanzen. Hier wird als Rezeptor R₃ ein Konjugat aus einem Rezeptor, der eine Bindungsstelle für die zu bestimmende Substanz aufweist und einem Partner P verwendet.

Variante 1d) ist geeignet zum Nachweis von monovalenten Substanzen. Als Rezeptor R₃ wird ein Konjugat verwendet, das zwei Bindungsstellen für die zu bestimmende Substanz und einem Partner P aufweist.

### Beispiel 1

a) Herstellung von Streptavidin-Latex
   Streptavidin in einer Konzentration von 2 mg/ml wird in 15 mMol/l Imidazolpuffer, pH 7,5, 100 mMol/l NaCl zusammen mit Chlormethylstyrolpartikeln (Latex, d = 70 nm, entsprechend US-A-4,703,018) in einer Konzentration von 2 Gew.-% 24 Stunden bei 55°C inkubiert und gerührt. Nachdem die Reaktionsmischung während 60 Minuten bei 20 000 Upm zentrifugiert worden ist, wird der Überstand abdekantiert und der Niederschlag in 200 mMol/l Glycin-Puffer, pH 7,5, mit 0,5 % Rinderserumalbumin aufgenommen. Durch entsprechende Verdünnung wird ein 1 Gew.-%iges Streptavidin-Latexreagenz hergestellt.
b) Herstellung von Hapten-Biotin-Konjugaten
   Hierzu wird n-Butyloxycarbonyl-tetrajodothyronin und 1-Methyl-3-(3′-carboxypropyl)-xanthin (DE-A 28 05 961) über Pentamethylendiamin mit Biotin gekoppelt, wie in Eur. J. Biochem. 131 (1980) 333-338 beschrieben. Man erhält T₄-Biotin-Konjugat. In analoger Weise wird Theophyllin an Biotin gekoppelt.

### Beispiel 2

### Bestimmung von T₄

Ein Reagenz, bestehend aus:
900 »l 0,1 mol/l Phosphatpuffer, pH 7,5, mit 2 %
Polyethylenglykol 40000
20 »l Probe (wäßrige T₄-Standards)
20 »l einer 0,1 mg/ml-Lösung eines polyklonalen
Antikörpers gegen T₄
20 »l 1 %iges Latex-Reagenz (Beispiel 1)
wird 5 Minuten bei 37°C inkubiert und anschließend 20 »l eines Konjugats aus T₄ und Biotin der Konzentration 10⁻⁶ mol/l zugegeben.

Es wurde die Kinetik der Trübungszunahme gemessen und die Extinktionsänderung bei 405 nm/Zeiteinheit (5 Minuten) bestimmt. Dabei ergaben sich folgende Meßwerte:

**Tabelle I**

| Probe/ng/ml T₄/ | mE/5 min |
|---|---|
| 0 | 620 |
| 50 | 500 |
| 100 | 410 |
| 200 | 230 |
| 500 | 60 |

### Beispiel 3

### Bestimmung von Theophyllin

Es wird ein Reagenz hergestellt durch Mischen von:
300 »l 100 mMol/l Tris-Puffer, pH 7,5
7 »l 1%iges Latex-Reagenz (Beispiel 1),
7 »l polyklonalar Antikörper gegen Theophyllin (Fab₂ "Konzentration 5 mg/ml")
und 5 Minuten bei 37°C inkubiert. Anschließend werden 5 x 10⁻⁶ Mol/l eines Konjugats aus Theophyllin und Biotin zugegeben und die Extinktionsänderung bei 450 nm bei einer Zeitdifferenz von 4,2 Minuten bestimmt. Die Ergebnisse sind in Tabelle II zu sehen.

**Tabelle II**

| Probe uM/l Theophyllin | mE/4,2 min |
|---|---|
| 0 | 1050 |
| 0,5 | 880 |
| 2,5 | 800 |
| 5 | 550 |
| 12,5 | 320 |
| 25 | 230 |
| 50 | 70 |
| 125 | 40 |

### Beispiel 4

### Vergleich mit dem Stand der Technik

Das erfindungsgemäße Verfahren wurde mit einem Verfahren, analog EP-A-0 073 611, verglichen. Als beschichteter "Träger" wurde Streptavidin verwendet, welcher 4 T₄-Moleküle pro "Träger" enthält.

Die Herstellung dieses "Trägers" erfolgte, indem 20 »l eines Konjugats aus T₄ und Biotin (Konzentration 10⁻⁴ Mol/l) mit 20 »l Streptavidin (Konzentration 2,5 x 10⁻⁵ Mol/l) zusammengegeben wurden und 30 Minuten inkubiert wurde. Nach Beendigung der Reaktion enthielt dieses Reagenz T₄, welches über Biotin an Streptavidin gekoppelt war.

### Durchführung des Vergleichsversuchs:

820 »l 50 mMol Phosphatpuffer, pH 7,5, + 4 % Polyethylenglykol 40 000 wurden mit 40 »l des wie oben beschrieben hergestellten Streptavidinreagenzes zusammengegeben und eine Probe (0 Mol/l T₄) zugegeben. Gleichzeitig wurden 100 »l einer Lösung von 3 mg/l Antikörper gegen polyklonalen Antikörper gegen T₄ zugegeben und die Extinktionsänderung pro 3 Minuten bei 340 nm verfolgt.

### Erfindungsgemäßes Verfahren, Variante A:

820 »l 50 mMol/l Phosphatpuffer, pH 7,5, + 4 % Polyethylenglykol 40 000 wurden mit 20 »l Konjugat aus T₄ und Biotin (Konzentration 10⁻⁴ Mol/l in Methanol) und 100 »l polyklonalen Antikörper gegen T₄ (3 mg/ml) zusammengegeben und 5 Minuten inkubiert.

Nach Zugabe von 20 »l Streptavidin (Konzentration 2,5 x 10⁻⁵ Mol/l) wird die Extinktionsänderung pro 3 Minuten bei 340 nm verfolgt.

### Erfindungsgemäßes Verfahren, Variante B:

820 »l 50 mMol/l Phosphatpuffer, pH 7,5, + 4 % Polyethylenglykol 40 000 werden mit 20 »l Streptavidin (2,5 x 10⁻⁵ Mol/l) in 200 mMol Phosphatpuffer, pH 7,5, und 100 »l polyklonalen Antikörper gegen T₄ (3 mg/ml ₎ zusammengegeben und 5 Minuten bei 37°C inkubiert. Nach Zugabe von 20 »l Konjugat aus T₄ und Biotin (Konzentration 10⁻⁴ Mol/l) wird die Extinktionsänderung pro 3 Minuten bei 340 nm verfolgt.

### Die Ergebnisse des Vergleichsversuchs sind:

Vergleich: 35 mE
Erfindungsgemäßes Verfahren, Variante A: 646 mE
Erfindungsgemäßes Verfahren, Variante B: 414 mE
Es zeigt sich, daß unter analogen Bedingungen mit dem erfindungsgemäßen Verfahren eine höhere Empfindlichkeit gegenüber dem Verfahren nach dem Stand der Technik erhalten werden kann.

## Patentansprüche

1. Verfahren zur Bestimmung einer spezifisch bindefähigen Substanz durch Inkubation der Probelösung mit mindestens drei Rezeptoren R₁,R₂ und R₃, von denen R₁ und R₂ miteinander bindefähig sind und R₃ mit der zu bestimmenden Substanz spezifisch bindefähig ist und Messung der bei der Reaktion auftretenden Agglutination,
**dadurch gekennzeichnet,**
daß als Rezeptor R₁ ein Konjugat aus einem Partner eines spezifisch bindenden Paares P und einer Substanz S, die der zu bestimmenden Substanz entspricht oder ein Derivat davon ist und mindestens ein Epitop der zu bestimmenden Substanz aufweist, wobei R₁ nur eine Bindungsstelle für R₂ besitzt, als R₂ ein Rezeptor, der mindestens zwei Bindungsstellen für P aufweist aber kein Erythrozyt ist und als R₃ ein Rezeptor, der mindestens zwei Bindungsstellen aufweist, von denen mindestens eine spezifisch mit einem Epitop der zu bestimmenden Substanz bzw. von S bindet, verwendet wird und R₂ gleichzeitig mit den Rezeptoren R₁ und R₃ oder nach einem bestimmten Zeitraum zugegeben wird oder R₂ und R₃ vorgemischt werden und nach Zugabe der Probe R₁ zugesetzt wird.

2. Verfahren nach Anspruch 1,
**dadurch gekennzeichnet,**
daß als R₃ ein Rezeptor verwendet wird, der eine zweite Bindungsstellte hat, die spezifisch mit einem Epitop der zu bestimmenden Substanz bzw. von S bindet.

3. Verfahren nach Anspruch 1,
**dadurch gekennzeichnet,**
daß als R₃ ein Rezeptor verwendet wird, der eine zweite Bindungsstelle in Form des ebenfalls für R₁ verwendeten Partners P aufweist, wobei die zu bestimmende Substanz nur eine einzige Bindungsstelle für Rezeptor R₃ aufweist.

4. Verfahren nach Anspruch 3,
**dadurch gekennzeichnet,**
daß man eine Substanz bestimmt, die ein einziges, mit R₃ bindefähiges Epitop aufweist und daß als R₃ ein Konjugat aus einem Partner P eines spezifisch bindenden Paares und einer Substanz, die eine Bindungsstelle aufweist, die spezifisch mit einem Epitop der zu bestimmenden Substanz bzw. von S bindet, verwendet.

5. Verfahren nach Anspruch 3,
**dadurch gekennzeichnet,**
daß eine Substanz bestimmt wird, die ein einziges mit R₃ bindefähiges Epitop aufweist und daß als R₃ ein Konjugat verwendet wird aus einem Partner P eines spezifisch bindenden Paares und einem Rezeptor, der zwei Bindungsstellen für das Epitop der zu bestimmenden Substanz bzw. S aufweist.

6. Verfahren nach einem der vorhergehenden Ansprüche,
**dadurch gekennzeichnet,**
daß als Rezeptor R₁ ein Konjugat aus P und der zu bestimmenden Substanz verwendet wird.

7. Verfahren nach einem der vorhergehenden Ansprüche,
**dadurch gekennzeichnet,**
daß als Rezeptor R₂ ein partikulärer Träger verwendet wird, an dem eine Vielzahl der zu P komplementären Partner des spezifisch bindenden Paares gebunden sind.

8. Reagenz zur Bestimmung einer spezifisch bindefähigen Substanz nach einem der Ansprüche 1 bis 7,
**dadurch gekennzeichnet,**
daß es Rezeptor R₁, der ein Konjugat aus einem Partner eines spezifisch bindenden Paares P und einer Substanz S, die der zu bestimmenden Substanz entspricht oder ein Derivat davon ist und mindestens ein Epitop der zu bestimmenden Substanz aufweist, wobei R₁ nur eine Bindungsstelle für R₂ besitzt, Rezeptor R₂, der mindestens zwei Bindungsstellen für P aufweist aber kein Erythrozyt ist und Rezeptor R₃, der mindestens zwei Bindungsstellen aufweist, von denen mindestens eine spezifisch mit einem Epitop der zu bestimmenden Substanz bzw. von S bindet, enthält.

9. Reagenz nach Anspruch 8,
**dadurch gekennzeichnet,**
daß es R₁, R₂ und R₃ physikalisch getrennt voneinander enthält, R₁ ein Konjugat aus der zu bestimmenden Substanz und einem Partner eines spezifisch miteinander bindenden Paares ist und R₂ einen agglutinierbaren Rezeptor, der eine Vielzahl der anderen Partner des spezifisch bindenden Paares P aufweist, darstellt.

10. Reagenz nach einem der Ansprüche 8 oder 9,
**dadurch gekennzeichnet,**
daß es als Rezeptor R₃ ein Konjugat aus einem Partner P eines spezifisch bindenden Paares und einer Substanz, die eine Bindungsstelle aufweist, die spezifisch mit einem Epitop der zu bestimmenden Substanz bzw. von S bindet, enthält.

## Claims

1. Process for the determination of a specifically bindable substance by incubation of the sample solution with at least three receptors R₁, R₂ and R₃, of which R₁ and R₂ are bindable with one another and R₃ is specifically bindable with the substance to be determined and measurement of the agglutination taking place in the case of the reaction, characterised in that, as receptor R₁, there is used a conjugate of a partner of a specifically binding pair P and of a substance S which corresponds to the substance to be determined or is a derivative thereof and has at least one epitope of the substance to be determined, whereby R₁ possesses only one binding site for R₂, as R₂ a receptor is used which has at least two binding sites for P but is not an erythrocyte and as R₃ a receptor is used which has at least two binding sites, of which at least one binds specifically with an epitope of the substance to be determined or of S, respectively, and R₂ is added simultaneously with the receptors R₁ and R₃ or after a definite period of time or R₂ and R₃ are premixed and R₁ is added after addition of the sample.

2. Process according to claim 1, characterised in that, as R₃, a receptor is used which has a second binding site which binds specifically with an epitope of the substance to be determined or of S, respectively.

3. Process according to claim 1, characterised in that, as R₃, a receptor is used which has a second binding site in the form of the partner P also used for R₁, whereby the substance to be determined has only a single binding site for receptor R₃.

4. Process according to claim 3, characterised in that one determines a substance which has a single epitope bindable with R₃ and that, as R₃, there is used a conjugate of a partner P of a specifically binding pair and of a substance which has a binding site which binds specifically with an epitope of the substance to be determined or of S, respectively.

5. Process according to claim 3, characterised in that a substance is determined which has a single epitope bindable with R₃ and that,as R₃, a conjugate is used of a partner P of a specifically binding pair and a receptor which has two binding sites for the epitope of the substance to be determined or S, respectively.

6. Process according to one of the preceding claims, characterised in that, as receptor R₁, a conjugate is used of P and of the substance to be determined.

7. Process according to one of the preceding claims, characterised in that, as receptor R₂, a particulate carrier is used to which are bound a plurality of the partners of the specifically binding pair complementary to P.

8. Reagent for the determination of a specifically bindable substance according to one of claims 1 to 7, characterised in that it contains receptor R₁ which is a conjugate of a partner of a specifically binding pair P and of a substance S which corresponds to the substance to be determined or is a derivative thereof and has at least one epitope of the substance to be determined, whereby R₁ possesses only one binding site for R₂, receptor R₂ which has at least two binding sites for P but is not an erythrocyte and receptor R₃ which has at least two binding sites of which at least one binds specifically with an epitope of the substance to be determined or of S, respectively.

9. Reagent according to claim 8, characterised in that it contains R₁, R₂ and R₃ physically separated from one another, R₁ is a conjugate of the substance to be determined and a partner of a pair specifically binding with one another and R₂ represents an agglutinisable receptor which has a plurality of the other partner of the specifically binding pair P.

10. Reagent according to one of claims 8 or 9, characterised in that, as receptor R₃, it contains a conjugate of a partner P of a specifically binding pair and of a substance which has a binding site which binds specifically with an epitope of the substance to be determined or of S, respectively.

## Revendications

1. Procédé de détermination d'une substance capable de se lier spécifiquement par incubation de la solution échantillon avec au moins trois récepteurs R₁, R₂ et R₃, parmi lesquels R₁ et R₂ sont capables de se lier l'un à l'autre et R₃ est capable de se lier spécifiquement à la substance à déterminer et mesure de l'agglutination qui se produit lors de la réaction, caractérisé en ce que l'on utilise comme récepteur R₁ un conjugué constitué par un partenaire d'une paire qui se lie spécifiquement P et par une substance S qui correspond à la substance à déterminer ou qui en est un dérivé et qui présente au moins un épitope de la substance à déterminer, R₁ ne possédant qu'un site de liaison pour R₂, comme R₂ un récepteur qui présente au moins deux sites de liaison pour P mais qui n'est pas un érythrocyte et comme R₃ un récepteur qui présente au moins deux sites de liaison parmi lesquels l'un au moins se lie spécifiquement à un épitope de la substance à déterminer ou de S, et l'on ajoute R₂ en même temps que les récepteurs R₁ et R₃ ou après une durée déterminée ou bien on prémélange R₂ et R₃ et on ajoute R₁ après addition de l'échantillon.

2. Procédé selon la revendication 1, caractérisé en ce que l'on utilise comme R₃ un récepteur qui a un second site de liaison qui se lie spécifiquement à un épitope de la substance à déterminer ou de S.

3. Procédé selon la revendication 1, caractérisé en ce que l'on utilise comme R₃ un récepteur qui présente un second site de liaison sous forme du partenaire P utilisé aussi pour R₁, la substance à déterminer ne présentant qu'un seul site de liaison pour le récepteur R₃.

4. Procédé selon la revendication 3, caractérisé en ce que l'on détermine une substance qui présente un seul épitope, capable de se lier à R₃, et en ce que l'on utilise comme R₃ un conjugué constitué par un partenaire P d'une paire qui se lie spécifiquement et par une substance qui présente un site de liaison qui se lie spécifiquement à un épitope de la substance à déterminer ou de S.

5. Procédé selon la revendication 3, caractérisé en ce que l'on détermine une substance qui comporte un seul épitope, capable de se lier à R₃, et en ce que l'on utilise comme R₃ un conjugué constitué par un partenaire P d'une paire qui se lie spécifiquement et par un récepteur qui présente deux sites de liaison pour l'épitope de la substance à déterminer ou de S.

6. Procédé selon l'une des revendications précédentes, caractérisé en ce que l'on utilise comme récepteur R₁ un conjugué constitué par P et par la substance à déterminer.

7. Procédé selon l'une des revendications précédentes, caractérisé en ce que l'on utilise comme récepteur R₂ un support particulaire auquel sont liés une multiplicité de partenaires, complémentaires de P, de la paire qui se lie spécifiquement.

8. Réactif pour la détermination d'une substance capable de se lier spécifiquement selon l'une des revendications 1 à 7, caractérisé en ce qu'il contient un récepteur R₁ qui est un conjugué constitué par un partenaire d'une paire qui se lie spécifiquement P et par une substance S qui correspond à la substance à déterminer ou qui en est un dérivé et qui présente au moins un épitope de la substance à déterminer, R₁ ne présentant qu'un site de liaison pour R₂, un récepteur R₂ qui présente au moins deux sites de liaison pour P mais qui n'est pas un érythrocyte et un récepteur R₃ qui présente au moins deux sites de liaison parmi lesquels l'un au moins se lie spécifiquement à un épitope de la substance à déterminer ou de S.

9. Réactif selon la revendication 8, caractérisé en ce qu'il contient R₁, R₂ et R₃ physiquement séparés les uns des autres, R₁ est un conjugué constitué par la substance à déterminer et par un partenaire d'une paire qui se lie mutuellement spécifiquement et R₂ représente un récepteur agglutinable qui présente une multiplicité des autres partenaires de la paire qui se lie spécifiquement P.

10. Réactif selon l'une des revendications 8 ou 9, caractérisé en ce qu'il contient comme récepteur R₃ un conjugué constitué par un partenaire P d'une paire qui se lie spécifiquement et par une substance qui présente un site de liaison qui se lie spécifiquement à un épitope de la substance à déterminer ou de S.
